# EUROPEAN PATENT SPECIFICATION

(11) **EP 3 218 032 B1**
(45) Date of publication and mention of the grant of the patent: **31.07.2019**
(21) Application number: 15790210.7
(22) Date of filing: 27.10.2015
(51) Int. Cl.: A61M 11/00, A61M 15/00, B29C 45/26, B05B 1/26, A61M 15/08, A61F 9/00, B05B 1/34

(54) **LOW COST IMPINGING JET NOZZLE**
KOSTENGÜNSTIGE STOSSSTRAHLDÜSE
BUSE À JETS DE COLLISION À FAIBLE COÛT

(30) Priority: 14.11.2014 GB 201420266
(43) Date of publication of application: 20.09.2017
(73) Proprietor: TTP PLC, Royston, Hertfordshire SG8 6EE (GB)
(72) Inventor: SELBY, Robert Gordon Maurice, Melbourn Herts. SG8 6DT (GB); STRANGE, Daniel Geoffrey Tyler, Cambridge CB4 2DJ (GB); RICHARDSON, Will, Royston Herts. SG8 9TS (GB)
(74) Representative: Gill Jennings & Every LLP
(86) International application number: PCT/GB2015/053221
(87) International publication number: WO 2016/075433

(56) References cited:
- EP-A2- 0 466 157
- WO-A1-2012/092688
- GB-A- 2 466 631
- US-A1- 2004 164 186

## Description

### Background

Aerosols are highly effective and user-friendly methods of delivering pharmaceutical ingredients to the lungs, nose, and eyes. Delivery is targeted, with fast uptake. Aerosols are also simple for users to apply without direct user contact with tissue, avoiding many of the complications from applying topical medicines such as eye drops.

Current low-cost aerosols (e.g. nasal pump sprays) typically eject a fast moving spray with a large fraction of large droplets that splash and dribble on impact, resulting in a poor user experience, There is a need for a low cost aerosol device (<£0.50) that produces a gentle, slow moving (<10 m/s) plume with a narrow droplet size distribution of 20 - 30 µm, with low flow rates (∼50 µl/s). It must be possible to manufacture the device in large quantities (millions per year), and the device must be robust to manufacturing tolerances. Such a device would have practical use for the aerosolisation of medicinal formulations (e.g. nasal, ophthalmic, sprays, topical) and consumer formulations (e.g. fragrances, sunscreen).

A slow mist can be produced by forcing liquid at high pressures through two holes such that the liquid jets impinge outside the nozzle and aerosolise. Such slow mists can be formed by using an injection moulded nozzle such as that disclosed in US5358179 (1994) or US7219849. However, it is difficult to reliably form holes less than 100 µms using injection moulding and as result the flow rate through such nozzles is orders of magnitude higher than what is desired for consumer health applications.

There is a need for a low cost means of reliably forming an impinging jet nozzle with hole sizes less than 100 µm. The axes of the holes must be accurately aligned and the hole exits must be precisely formed, such that when liquid is forced through the holes under pressures of greater than 1 bar, the resultant jets consistently intersect to form a slow moving aerosol. Furthermore, the hole walls should be relatively smooth to discourage deposits and subsequent blocking. Ideally, the nozzle should comprise a single part, to minimise assembly steps and BOM costs. The nozzle should be robust to pressures greater than 1 bar (and potentially up to 300 bar). It should made from a materials that exhibit reasonable chemical and moisture stability such that it can be exposed to many of the chemical solvents found in medicinal formulations without degradation in performance.

WO2009090084 introduces a method of forming an impinging jet nozzle with holes of 5 to 15 µm, whereby the holes are laser drilled perpendicularly into a flat plate. The flat plate is then deformed after manufacture such that the axes of the holes intersect each other. Although this is relatively simple method of manufacture, the thin sheet must be supported in order to connect it with the rest of the fluid feed and to prevent it permanently deforming under high pressure. A 7 mm diameter, 100 µm thick steel circular nozzle plate supported only at the edges will plastically deform at pressures as low as 6 bar. Hence, such a nozzle must be held in a nozzle holder, typically comprised of at least two injection moulded parts and an elastomeric face seal (e.g. US 7,621,266). This assembly must then still be sealed to the rest of the device. Furthermore, the manufacturing method is likely to be sensitive to manufacturing tolerances. The process is subject to variation from the shape of the deep drawing tool, the shape of the thin mesh, the position of the laser drilled holes within the thin mesh, the placement of the mesh within the deep drawing tools, and the amount of spring back after drawing, which will be affected by material and process properties GB 2466631 discloses in figure 6 an atomising element with a region housing nozzles and channels being in the form of concave dome such that jets converge and collide to form droplets with a small diameter.

The following invention proposes a number of forms of impinging jet nozzles with hole sizes less than 100 µm, that are low cost, tolerance insensitive, and require few assembly steps.

### Summary of the invention

The invention relates to a spray device and method(s) of forming such a device as set out in the accompanying claims.

The present invention provides a spray device according to claim 1.

For circular holes, the hydraulic diameter will be the diameter of the holes.

Preferably, the axes of the holes are orthogonal to the external surface of the thin walled regions as this typically produces the cleanest and most precise hole formation, although some deviation is possible with 35 degrees either side of orthogonal.

The nozzle assembly is preferably formed using a polymer, that has a melt flow index of at least 0.15g/10min, preferably 3g/10min, and ideally 20g/10min and an absorption coefficient greater than at least 0.001 µm-1, preferably 1 µm-1, and ideally 20 µm-1 ¬ at at least one wavelength less than 370 nm.

The nozzle assembly may be formed using a polymer doped with additives that enhance the absorption of laser energy.

The laser drilled holes are preferably formed using an Excimer laser and are preferably formed through the thin-walled sections.

The laser drilled holes may also be formed using a solid state laser with a harmonic generator unit, such as a frequency tripled Nd:YAG.

The thick wall sections preferably provide the sealing interface to the rest of the device and/or provide handling features for manufacture.

The invention also provides method of forming a spray device according to claim 9.

### Detailed Description

Figure 1 is an image of an impinging jet nozzle, formed according to the present invention, by laser drilling two holes into a moulded plastic part with thick and thin walls.
Figure 2 is a side cross-sectional view of an impinging jet nozzle, formed according to the present invention, by moulding a nozzle with two holes formed using a shaped mould pin and then plugging the wider section of the hole, such that only a small hole remains.
Figure 3 is a side cross-sectional view of an impinging jet nozzle, according to the present invention, formed by combining an outer component containing, a small moulded groove with a blade or plug components such that two small impinging holes are formed on assembly.
Figure 4 is a side cross-sectional view of an impinging jet nozzle, according to the present invention, formed by combining an outer nozzle with a blade or plug component, where the blade or plug component contains a small moulded channel, such that two small impinging holes are formed on assembly.
Figure 5 shows an impinging jet nozzle, according to the present invention, formed by combining an outer component with a conical plug component, where the outer component contains a small moulded channel, such that two small impinging holes are formed on assembly.
Figure 6 shows an impinging jet nozzle, according to the present invention, formed by combining an outer component with a conical plug component, where the outer component contains a small moulded channel, such that two small impinging holes are formed on assembly.
Figure 7 shows an impinging jet nozzle, according to the present invention, formed by combining an outer component with a conical plug component, where the conical plug contains a small moulded channel, such that two small impinging holes are formed on assembly.
Figure 8 shows an impinging jet nozzle, according to the present invention, containing a recess to hold a filter component.
Figure 9 shows an impinging jet nozzle, according to the present invention, where each hole is fed from a separate fluid feed, with a separate filter component.
Figure 10 shows an impinging jet nozzle, where the fluid feed narrows towards the exit in order to facilitate the shear thinning of viscous thixotropic liquids.
Figure 11 shows an impinging jet nozzle, where comprising channels that narrow towards the exit in order to facilitate the shear thinning of viscous thixotropic liquids.
Figure 12 a graph of viscosity plotted against time for a constant sheer stress applied to a thixotropic PEG based formulation.

### A single plastic moulded part with laser drilled holes

Plastic injection moulding is a common process used for manufacturing medical device components. In this process, molten polymer is pumped at high pressure into a moulding tool, wherein the polymer is cooled, and then ejected from the tool. Parts with complex shapes can be manufactured cheaply in large volumes.

Laser drilling of a polymer uses a high intensity laser to ablate material from a target region. When the beam hits the target surface, a combination of photochemical decomposition and thermal processes take place. Holes with diameters as small as 1 µm can be rapidly drilled. The process is also cost effective in high volumes.

An impinging jet nozzle can be formed by injection moulding a single plastic part and then laser drilling two or more holes into the part that impinge by design to form a slow mist. This method offers several advantages over drilling and deforming a membrane and then integrating it into an assembly. Firstly, an injection moulded plastic can have integrated large features that can withstand high pressures and user handling, and be used to seal to the rest of the device without an additional nozzle holder. Secondly, the part can contain features such that it can be easily aligned relative to the laser. By laser drilling the holes into a single part, perpendicular to the outlet surface, the tolerances are defined solely by the position of the laser relative to the part.

While an injection moulded polymer impinging jet nozzle with laser drilled holes is attractive for its cost of goods and low assembly costs, it is difficult to reliably implement in practice. The placement of the laser drilled holes must be consistent, and furthermore the holes must be well formed and highly reproducible otherwise the nozzles will produce irregular jets of poorly controlled diameter that deviate from their intended trajectory and do not impinge to form a slow mist. The injection moulded part can be designed in such a way to facilitate this, with certain requirements onto the laser set-up used, the material properties of the polymer, and the geometry of the moulded plastic part.

In order to achieve a good hole quality in a polymer, it is desirable to use an Excimer laser. Typical chemical bonds in polymers have bond energies in the region of 3-8eV (for example, C-C, C=O). By comparison, the photon energies of various lasers are shown in table 1. The first two lasers in the table (CO₂ and YAG) produce photons clearly below the bond energy of polymers. This typically results in a thermal ablation process with a large heat affected zone (HAZ), which can cause distortion and unfavourable changes to the mechanical properties of the surrounding polymer. By contrast, the last 3 lasers in the table (Excimer lasers) all produce photons around or above the bond energy of polymers. This means that a much greater proportion of the energy absorbed by the polymer will be used in breaking chemical bonds, resulting in less heating of the surrounding polymer and a smaller HAZ. Alternatively, a solid state Nd:YAG laser may be used with a harmonic generator unit, in order to triple or quadruple the frequency, and drill at wavelengths of 370 nms and below.

**Table 1: Photon energy available for typical lasers.**

| **Laser type** | **Wavelength (nm)** | **Photon energy (eV)** |
|---|---|---|
| CO₂ | 10600 | 0.12 |
| Nd:Yag | 1064 | 1.2 |
| XeCl | 308 | 4 |
| KrF | 248 | 5 |
| ArF | 193 | 6.4 |

Furthermore, it is important that a polymer with a high absorption coefficient is used. A larger α value results in the absorption of photons being concentrated in a smaller region at the surface of the polymer. It has been found experimentally that polymers with a higher absorption coefficient are better suited to laser drilling clean holes (see for example; Martin F Jensen, Laser micromachining of polymers (PhD thesis), p73). Polymers with poor absorption, such as PTFE at 248nm, will tend to produce non-uniform and highly variable features. For reliable drilling to be performed, a polymer is required with an absorption coefficient of >0.001 µm⁻¹ at the wavelength produced by the laser.

In terms of geometry, the sheets of material being drilled into are preferably relatively thin (<200 µm) - the deeper the hole being drilled the more likely it is the ablated material will redeposit in the hole, and the radiation will be attenuated by the edges of the hole. It is also likely that reflections laser beam will reflect off the hole walls, causing further deterioration in hole quality. Furthermore, the laser drilled holes should preferably be drilled perpendicular to the external surface. Holes laser drilled at an oblique angle are characterised by uneven profiles (Wu et al., Effects of excimer laser illumination on microdrilling into an oblique polymer surface. Optics and Lasers in Engineering) and are unlikely to produce two jets that reliably collide.

Although it is desirable to have thin wall sections to reliably laser-drill holes, these thin wall sections are preferably supported by thicker wall sections (> 200 µm) in the injection moulded part in order to ensure that the part can withstand the large generated pressures without substantial deformation. Moulding thin wall sections in an otherwise-thick part can be difficult to achieve using injection moulding. As the polymer flows through the tool, the melt in contact with the walls of the tool will cool and solidify very quickly, and the polymer will progressively cool over time, gradually freezing further from the wall. This means that thin sections of the tool cavity (and the resulting plastic part) will freeze first. Depending on the geometry of the part, some parts of the cavity will have frozen through the entire part thickness before the whole cavity has been filled. This can block the flow of the molten polymer, preventing the part from being completely formed. This phenomenon is commonly referred to as 'short shot'.

Short shot can be overcome in several ways. One of these is to increase the tool temperature, so that the polymer remains soft and inviscid for longer. However, this increases the time required to manufacture the part ('cycle time'), which increases production costs. An alternative is to increase the pressure at which the polymer is injected, though this is likely to result in increased flash. A third, preferred option is to change the selected polymer for one with a lower viscosity, so that it flows more quickly into the tool. This can allow the part to be fully filled more easily, without the negative aspects of higher tool temperature or packing- or hold-pressure.

In the context of injection moulding, the viscosity of a polymer is commonly defined by the Melt Flow Index (MFI), which is measured according to protocols set out in standards such as ASTM D1238 and ISO 1133. These tests typically involve applying a specified load to polymers at a specified temperature, and measuring the mass of material that can be pushed through a capillary of specified dimensions in a specified period of time. The flow through the capillary is limited by viscous effects, so a higher MFI indicates that the polymer has a lower viscosity. A typical result may be 18g/10min, such as quoted by Honam Petrochemical corp for their J-560S Polypropylene.

Only a small subset of polymers (without additives to improve laser drilling) have both MFIs and high absorption coefficients of wavelengths between 150 and 370 nm. Preferred polymers include polycarbonate, PMMA, nylon, ABS, or PET. Furthermore it is desirable that these polymers exhibit chemical and moisture stability to many of the chemical solvents found in medicinal formulations such that the nozzle can be used to spray these formulations without degradation in performance.

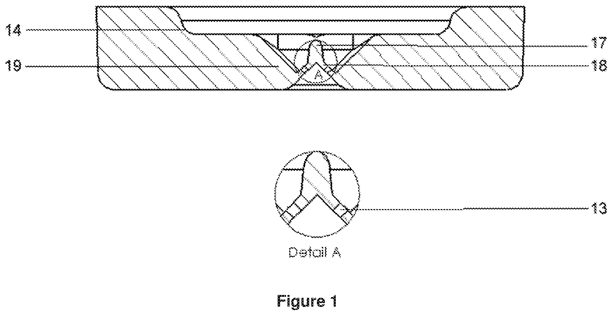

In light of the difficulties described above, the design of an injection moulded impinging jet nozzle with laser drilled holes that can be reliably formed is described. The nozzle is formed from a single injection moulded plastic part shown in **Figure 1****,** containing one or more thin wall sections <200 µm thick **(18)** into which holes **(13)** are laser drilled approximately perpendicular (±25 degrees) to the surface, using an Excimer laser. The thin wall section(s) are angled such that the axes of the holes intersect each other. These thin wall sections are preferably approximately square, i.e. have a substantially square projected area, and have a projected area of around 60,000µm², and are supported on all sides by a thick-walled region **(19, 17)** of the part. By having a small area, with a relatively small aspect ratio (preferably between 1 and 10 and ideally less than 4, defined by the ratio of unsupported length (longest side) of thin-walled region : wall thickness of the thin-walled region), large pressure differences in excess of 100bar can be sustained across the thin wall without plastic deformation of the part. Furthermore, the thick wall sections (>200 µm), prevent the thin wall sections deflecting by more than 5 degrees under pressures of 5 - 50 bar.

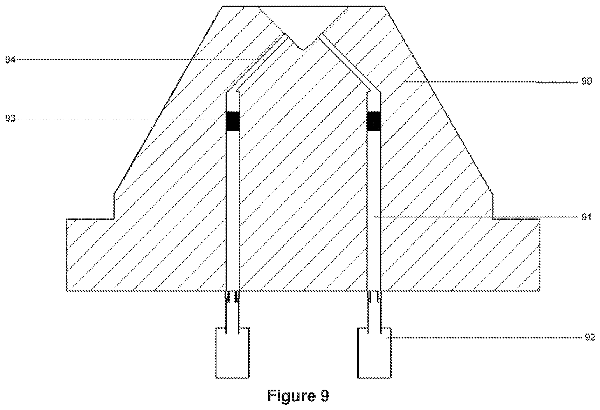

The thick wall sections **(19)** also provide a sealing interface to the rest of the aerosol device, such that the nozzle can be used without an additional nozzle holder assembly and provide alignment features for manufacture. The recess **(14)** provides a location for an optional filter elements as described in reference to **Figure 9** later in the text.

In order to create these nozzle features in an injection moulded part, the polymer is injected into through the thick-walled region of the part **(19),** such that that the polymer remains in a molten state in the thick-walled region, while the thin-walled region is completely filled. The nozzle is manufactured using a polymer that has a melt flow index of greater than 0.15g/10min such that short shot problems are avoided, and absorption coefficient at the wavelength emitted by the chosen laser of > 0.001 µm⁻¹, such that the laser drilled holes are well-formed. Polycarbonate drilled with a krf laser is an example of a polymer and laser system that satisfies both of these requirements. Alternatively a polymer with an additive to improve laser drilling performance can also be used.

### Combined large and small moulded feature to support small feature during moulding

When moulding plastic components the minimum size of through holes is often limited by the structural properties of the mould pin needed to form the small hole. Below reasonable limit of 100 µm or more often 200 µm the challenge of avoiding mould pin breakage and achieving stability during moulding becomes problematic for volume production. The use of wire as a continuously replaceable mould pin can help with this challenge but it too has limitations, especially in the case of wanting to form two nozzles that have intersecting axes.

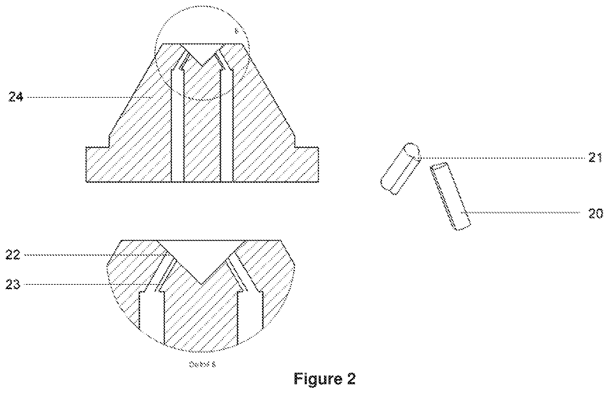

Therefore the use of a shaped mould pin such as that in **figure 2** offers an alternative, not covered by the invention. In this case the thicker section **(20)** provides the necessary support for the pin to withstand the moulding process reliably and the small feature **(21)** allows the formation of a narrow channel.

Following moulding if the wider section **(22)** is plugged by a ball, rod or similar solid object such as a short length of extruded rod or a moulded pin then this offers a means to embody two small holes formed by the exit of the channel **(23)** that are accurately placed and with aligned axes to facilitate impinging jets whilst using conventional moulding

### Grooves in plug or body

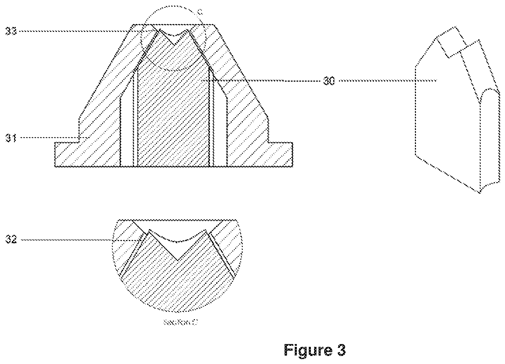

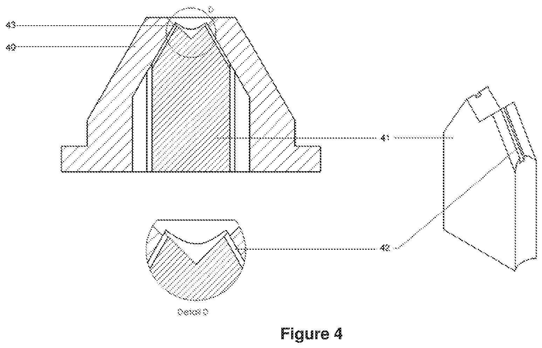

An alternative method not covered by the invention of forming narrow tubular features is to combine small features in one component as a groove or channel at the edge of a wider opening and then plug the wider opening with a separate component. **Figure 3** shows a nozzle body moulded **(31)** with a larger central slot which in turn has narrow channels or grooves at its edges (32). **The larger central slot is plugged by a separate component (30).** Similarly **Figure 4** shows an arrangement where small grooves are formed on the blade or plug component **(41).** In both **Figures 3** and **4** the combination of the blade or plugging component and the outer body allow formation of small closed fluid channels terminating in nozzles **(33)** and **(43)** respectively suitable for forming impacting jets and are well suited to conventional moulding methods.

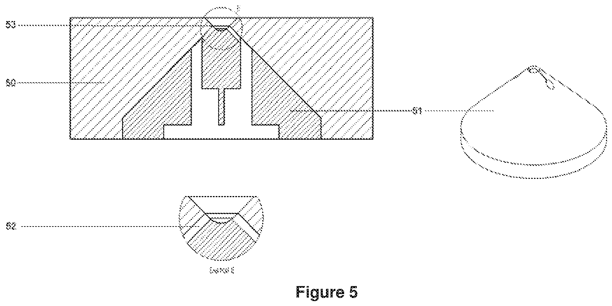

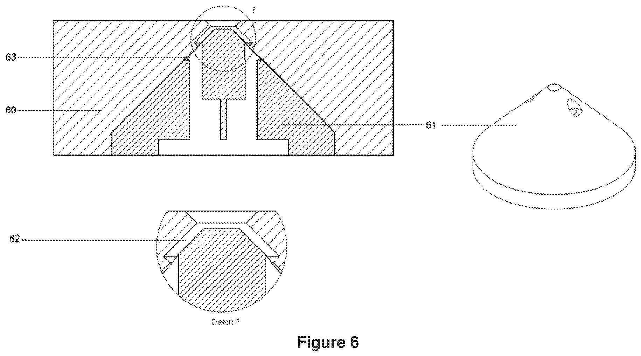

A further embodiment not covered by the invention using a similar principle replaces the flat central plug with one of a circular truncated conical form. In **Figure 5** the grooves needed to form the narrow channels are formed in the conical plug **(51)** and when this is pressed into place within the body **(50)** narrow and aligned nozzles can be formed. An advantage to the conical plug approach is that it is orientation independent for the assembly of the components **(50)** and **(51).** In a similar way to **Figures 3** and **4** the small groove features could be formed in either of the mating components. **Figure 6** shows an implementation where the groove features are formed in the body component **(60).**

In designing such components it can be advantageous to include all of the fluid feed sections in one or other component to eliminate the need for alignment of the two components. However by including wide openings such as an annular manifold **(64)** in the body component **(60)** in **Figure 6** such need for alignment can be eliminated..

### U Channels and narrow tubes

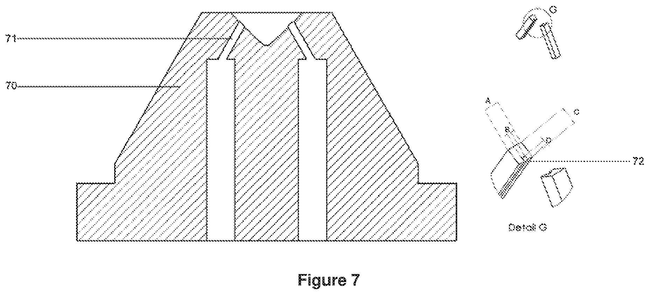

As an alternative not covered by the invention to moulding narrow features into the body component simple larger holes could be moulded such that when these are plugged by a secondary component a small hole is formed. This arrangement is shown in **Figure 7** and the shape of the holes **(71)** can be chosen to suit the material used to plug the holes. This offers the advantage of making the body component entirely suited to standard injection moulded volume production methods. The elements used to plug the holes can be of the form of a narrow tube with a fine inner diameter, such as could be formed by deep drawing stainless steel, or an extruded tube such as those used in HPLC pipework which is commercially available in outer diameter sizes including 0.36mm to 1.57mm with inner diameters including 25 µm, 50 µm, 65 µm and 75 µm. Alternatively the plugging element could be an open channel such as the form of a U channel **(72)** where the narrow groove will in combination with the hole in the body forms the required nozzles. Such an element could be moulded or extruded as the open structure is particularly well suited to extrusion.

### Filter

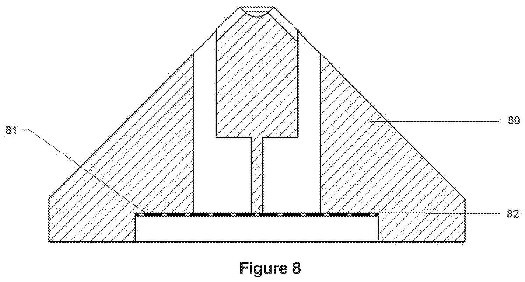

For reliable operation it is sometimes beneficial to include a filter component close to the exit nozzles to avoid contamination and subsequent blocking of the nozzles. It is further beneficial if such a filter is located a short distance along the fluid path from the exit nozzles. For the nozzle constructions seen in **Figures 3****,** **4****,** **5** **and** **6** provision of a recess in the body of the component allows a filter element to be pressed into place within the nozzle assembly. Such an arrangement is shown in **Figure 8** where **(80)** represents a generic version of the nozzle body as seen for the various options discussed in reference to figures including 3, 4, 5 and 6. The recess in the body **(80)** labelled **(81)** is suitable for receiving the filter element and **(82)** is the filter element. Optionally the filter element may incorporate a seal such as a compliant member moulded to the edge of the filter or a seal such as an O ring could be included between the filter and nozzle body. The filter element itself could be a fused metal filter such as a sintered filter block, a fused polymer filter or other filter elements well known in the art. Alternatively to suit this particular application a laser drilled membrane may be used as this provides a clean filter which does not need post manufacturing washing, provides a specific hole size to act as an absolute filter. Whereas a drilled membrane may ordinarily be overlooked for many filtering applications due to relatively low contamination tolerance in this controlled case this limitation is not a significant drawback.

### Dual fluid feed

There may be occasions when it would be beneficial to feed different fluids to each nozzle. In the case of a dual nozzle arrangement this would allow mixing of two different liquids that could not be stored together for stability or compatibility reasons but which would be beneficially delivered as a combined dose. Alternatively in a dual or multi nozzle arrangement the delivery of air or other gas may be used to alter the shape of the plume of delivered droplets to help optimise the plume to the intended use. For example to increase dispersion of the plume to cover a wider area or to help mix the plume with surrounding air and so help evaporation. By arranging the fluid feeds to be separate from each other in either the body or central plug features as shown schematically in **Figure 9** individual fluid connections can be made **(92)** to supply the nozzles **(94).** Filter elements could be added to the individual fluid paths **(93).**

### Shear Thinning

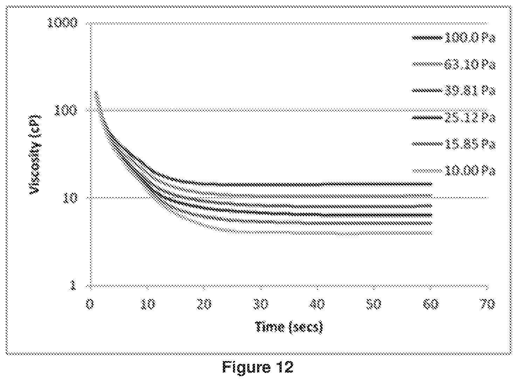

Many drug formulations and in particular topically applied formulations have high static viscosity but shear thin to a significant extent. For example, the viscosity of a PEG based formulation for a nasal spray can decrease by over 100 times when a shear stress is applied (**Figure 12**). The high static viscosity can prove problematic for spray devices which need relatively narrow channels, such as those described above, and high fluid speeds to form small droplets and so may have issues with achieving sufficient flow for successful start up. Shear thinning is particularly important for the impinging nozzles described in all the various examples due to the small hole sizes.

By arranging the fluid to be held away from the narrowest part of the fluid dispensing head and having a fluid path that gradually narrows towards the nozzle can resolve this issue. Once fluid is first acted on by the pressure generating mechanism it will start to move and start to shear thin. As the fluid moves to the narrower cross sectional areas the speed of movement will increase and the shear thinning will also increase.

One form of nozzle of reducing cross section is illustrated in **Figure (10****).** Here the fluid path gradually reduces in cross section so that fluid starting at the wider part of the fluid path is accelerated by conservation. As the fluid starts to move so the molecules align and the fluid starts to shear thin. By arranging the geometry such that the fluid shear thins progressively through its journey through the flow path the pressure needed to initiate flow will not be significantly higher than that needed to maintain the target flow rate once the fluid has shear thinned. This is achieved by the ratio of areas of the start and end of the fluid path and the rate of change such that there is sufficient time for the shear thinning to occur.

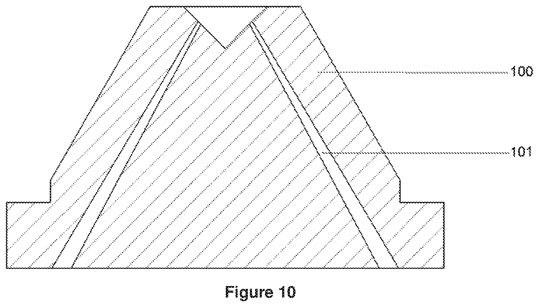

The narrowing cross section nozzles could be formed using the methods and/or structures described above in relation to Figures 1 to 9, especially when the nozzle tapers to the outlet as shown in Figure 10. Alternatively or additionally, narrowing channels could be formed by moulding or by the use of a separate component as described below.

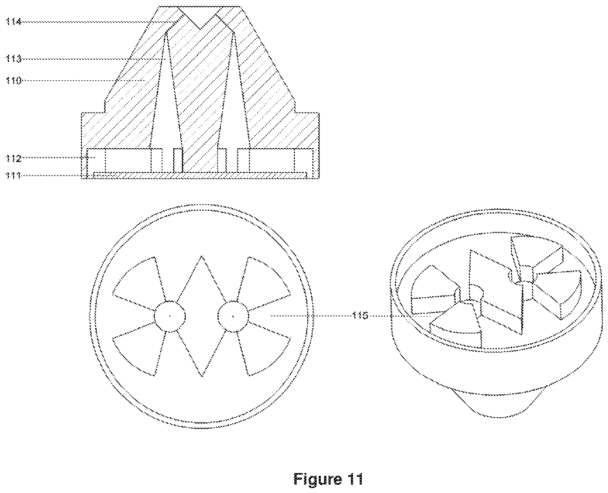

A further implementation **(****Figure 11****)** of the progressively narrowing fluid path can be implemented by construction of a nozzle head from a number of open channels **(115)** of reducing cross section which are covered and sealed by a second member **(111).** The second member **(111)** is connected to the fluid reservoir by means such as an annular manifold channel **(112)** or other relatively large cross sectional area fluid channel. In turn the, now sealed, channels **(115)** connect to tapered holes **(113)** which communicate with the exit nozzles **(114).** If the reducing areas channels formed by such a method are dry at the start of the spray then as the fluid is first acted on it will be accelerated from a wide cross sectional area to the narrow area. Again by designing the geometry appropriately the rate of acceleration can be tuned to match the shear thinning time behaviour of the fluid such that the progression of flow speed matches the shear thinning of the fluid.

## Claims

1. A spray device for generating a slow moving aerosol, whereby the aerosol is generated from at least two impinging jets and the jets are formed by forcing liquid through a single moulded plastic nozzle assembly, the device **characterised by**:
two or more thin walled sections (18) (typically <200 µm) which are each approximately square such as to have a substantially square projected area, where each of the thin wall sections (18) is supported on all sides by thick walled sections (17, 19) (typically >200 µm), and where an aspect ratio, which is defined by the ratio of length of a side of a respective thin walled section (18) to the wall thickness of the thin walled section (18), is between 1 and 10; and
at least two opposing holes (13), each of the holes (13) extending through a respective one of the thin walled sections (18), each of the holes (13) with hydraulic diameter of 5 µm to 100 µm (typically 30 µm) and an axis at an angle of between 55 and 125 degrees (preferably 90 degrees) to an external surface of the respective thin walled section (18), the thin walled sections (18) being angled such that the projected areas of the holes (13) at least partially intersect at the outlet side of the nozzle.

2. A spray device as in claim 1, wherein the nozzle assembly is formed using a polymer, that has a melt flow index of at least 0.15g/10min, preferably 3g/10min, and ideally 20g/10min and an absorption coefficient greater than at least 0.001 µm⁻¹, preferably 1 µm⁻¹ and ideally 20 µm⁻¹ at at least one wavelength less than 370 nm.

3. A spray device as in claim 1, wherein the nozzle assembly is formed using a polymer doped with additives that enhance the absorption of laser energy.

4. A spray device as in claim 1, wherein the holes (13) are laser drilled holes which are formed using an Excimer laser.

5. A spray device as in claim 1, wherein the holes (13) are laser drilled holes which are formed using a solid state laser with a harmonic generator unit.

6. A spray device according to any one of the preceding claims, wherein the thick wall sections (17, 19) provide the sealing interface to the rest of the device.

7. A spray device according to any one of the preceding claims, wherein the thick walled sections (17, 19) provide handling features for manufacture.

8. A spray device according to any one of the preceding claims, wherein the holes (13) have been formed by laser drilling through the thin walled sections (18).

9. A method of forming a spray device for generating a slow moving aerosol, whereby the aerosol is generated from at least two impinging jets and the jets are formed by forcing liquid through a single moulded plastic nozzle assembly, the method **characterised by** the steps of:
forming a single moulded plastic part comprising two or more thin walled sections (18) which are each approximately square such as to have a substantially square projected area, where each of the thin wall sections (18) is supported on all sides by thick walled sections (17, 19), and where an aspect ratio, which is defined by the ratio of length of a side of a respective thin walled section (18) to the wall thickness of the thin walled section (18), is between 1 and 10; and
laser drilling at least two opposing holes (13), each of the holes (13) extending through a respective one of the thin walled sections (18), each of the holes (13) with hydraulic diameter of 5 µm to 100 µm (typically 30 µm) and an axis at an angle of between 55 and 125 degrees (preferably 90 degrees) to an external surface of the respective thin walled section (18), the thin walled sections (18) being angled such that the projected areas of the holes (13) at least partially intersect at the outlet side of the nozzle assembly.

## Patentansprüche

1. Sprühvorrichtung zur Erzeugung eines sich langsam bewegenden Aerosols, womit das Aerosol ab zumindest zwei Stoßstrahldüsen erzeugt wird und die Strahlen durch Forcieren von Flüssigkeit durch eine einzelne Düsenanordnung aus Kunststoff geformt werden, wobei die Vorrichtung **gekennzeichnet ist durch**:
Zwei oder mehrere dünnwandige Abschnitte (18) (typisch <200 µm), die jeweils etwa quadratisch sind, wie beispielsweise eine im Wesentlichen quadratisch projizierte Fläche aufzuweisen, wobei jeder der dünnwandigen Abschnitte (18) auf allen Seiten **durch** dickwandige Abschnitte (17, 19) (typisch >200 µm) unterstützt wird, und wobei ein Aspektverhältnis, das durch das Verhältnis der Länge von einer Seite eines jeweiligen dünnwandigen Abschnitts (18) zur Wanddicke des dünnwandigen Abschnitts (18) definiert ist, zwischen 1 und 10 beträgt; und
zumindest zwei gegenüberliegende Löcher (13), wobei sich jedes der Löcher (13) **durch** einen jeweiligen der dünnwandigen Abschnitte (18), jedes der Löcher (13) mit hydraulischem Durchmesser von 5 µm bis 100 µm (typisch 30 µm) und einer Achse mit einem Winkel zwischen 55 und 125 Grad (vorzugsweise 90 Grad) zu einer Außenfläche des jeweiligen dünnwandigen Abschnitts (18) erstreckt, wobei die dünnwandigen Abschnitte (18) derartig abgewinkelt sind, dass sich die projizierten Flächen der Löcher (13) zumindest teilweise an der Austrittsseite der Düse schneiden.

2. Sprühvorrichtung nach Anspruch 1, wobei die Düsenanordnung unter Verwendung eines Polymers geformt ist, das einen Schmelzflussindex von zumindest 0,15g/10min, vorzugsweise 3g/10min und ideal 20g/10min und einen Absorptionskoeffizienten größer als zumindest 0,001 µm⁻¹, vorzugsweise 1 µm⁻¹ und ideal 20 µm⁻¹ bei zumindest einer Wellenlänge von weniger als 370 nm aufweist.

3. Sprühvorrichtung nach Anspruch 1, wobei die Düsenanordnung unter Verwendung eines Polymers geformt ist, das mit Zusatzmitteln dotiert ist, welche die Absorption von Laserenergie verbessern.

4. Sprühvorrichtung nach Anspruch 1, wobei die Löcher (13) lasergebohrte Löcher sind, die unter Verwendung eines Excimerlasers geformt sind.

5. Sprühvorrichtung nach Anspruch 1, wobei die Löcher (13) lasergebohrte Löcher sind, die unter Verwendung eines Festkörperlasers mit einer harmonischen Generatoreinheit geformt sind.

6. Sprühvorrichtung nach einem der vorhergehenden Ansprüche, wobei die dicken Wandabschnitte (17, 19) die Dichtungsschnittstelle zum Rest der Vorrichtung bereitstellen.

7. Sprühvorrichtung nach einem der vorhergehenden Ansprüche, wobei die dicken Wandabschnitte (17, 19) Handling-Merkmale für die Herstellung bereitstellen.

8. Sprühvorrichtung nach einem der vorhergehenden Ansprüche, wobei die Löcher (13) mittels Laserbohrung durch die dünnwandigen Abschnitte (18) geformt worden sind.

9. Verfahren zum Formen einer Sprühvorrichtung zur Erzeugung eines sich langsam bewegenden Aerosols, womit das Aerosol ab zumindest zwei Stoßstrahldüsen erzeugt wird und die Strahlen durch Forcieren von Flüssigkeit durch eine einzelne geformte Plastikdüsenanordnung geformt werden, wobei das Verfahren durch folgende Schritte gekennzeichnet ist:
Formen eines einzelnen Kunststoff-Formteils, das zwei oder mehrere dünnwandige Abschnitte (18) umfasst, die jeweils etwa derartig quadratisch sind, um eine im Wesentlichen quadratisch projizierte Fläche aufzuweisen, wobei jeder der dünnwandigen Abschnitte (18) auf allen Seiten durch dickwandige Abschnitte (17, 19) unterstützt wird, und wobei ein Aspektverhältnis, das durch das Verhältnis der Länge von einer Seite eines jeweiligen dünnwandigen Abschnitts (18) zur Wanddicke des dünnwandigen Abschnitts (18) definiert ist, zwischen 1 und 10 beträgt; und
Laserbohren von zumindest zwei gegenüberliegenden Löchern (13), wobei sich jedes der Löcher (13) durch einen jeweiligen der dünnwandigen Abschnitte (18), jedes der Löcher (13) mit hydraulischem Durchmesser von 5 µm bis 100 µm (typisch 30 µm) und einer Achse mit einem Winkel zwischen 55 und 125 Grad (vorzugsweise 90 Grad) zu einer Außenfläche des jeweiligen dünnwandigen Abschnitts (18) erstreckt, wobei die dünnwandigen Abschnitte (18) derartig abgewinkelt sind, dass sich die projizierten Flächen der Löcher (13) zumindest teilweise an der Austrittsseite der Düseanordnung schneiden.

## Revendications

1. Dispositif de pulvérisation pour la génération d'un aérosol à déplacement lent, l'aérosol étant généré à partir d'au moins deux jets incidents, les jets étant formés en forçant un liquide à travers un ensemble d'embout unique en plastique moulé, le dispositif étant **caractérisé par** :
deux ou plusieurs segments à paroi mince (18) (mesurant généralement < 200µm), chacun étant à peu près carré de façon à présenter une surface projetée substantiellement carrée, chacun des segments à paroi mince (18) étant supporté sur tous les côtés par des segments à paroi épaisse (17, 19) (mesurant généralement > 200µm), et un rapport de forme, défini par le rapport de la longueur d'un côté d'un segment à paroi mince (18) correspondant sur l'épaisseur de paroi du segment à paroi mince (18), étant compris entre 1 et 10 ; et
au moins deux trous opposés (13), chacun des trous (13) s'étendant à travers un segment correspondant des segments à paroi mince (18), chacun des trous (13) mesurant de 5µm à 100µm de diamètre hydraulique (généralement 30µm) et son axe se trouvant à un angle compris entre 55 et 125 degrés (de préférence 90 degrés) par rapport à une surface externe du segment à paroi mince (18), les segments à paroi mince (18) étant inclinés de sorte que les surfaces projetées des trous (13) se croisent au moins partiellement du côté de la sortie de l'embout.

2. Dispositif de pulvérisation selon la revendication 1, l'ensemble d'embout étant réalisé avec un polymère ayant un indice de fluidité d'au moins 0,15 g/10 min, de préférence 3 g/10 min, et idéalement 20g/10 min, et un coefficient d'absorption supérieur à au moins 0,001 µm⁻¹, de préférence 1 µm⁻¹, et idéalement 20 µm⁻¹, et au moins une longueur d'onde inférieure à 370 nm.

3. Dispositif de pulvérisation selon la revendication 1, l'ensemble d'embout étant réalisé avec un polymère dopé avec des additifs renforçant l'absorption de l'énergie laser.

4. Dispositif de pulvérisation selon la revendication 1, les trous (13) étant des trous percés au laser réalisés à l'aide d'un laser excimère.

5. Dispositif de pulvérisation selon la revendication 1, les trous (13) étant des trous percés au laser réalisés à l'aide d'un laser solide avec un générateur d'harmonique.

6. Dispositif de pulvérisation selon une quelconque des revendications précédentes, les segments à paroi épaisse (17, 19) formant une interface d'étanchéité pour le restant du dispositif.

7. Dispositif de pulvérisation selon une quelconque des revendications précédentes, les segments à paroi épaisse (17, 19) formant des éléments de manutention pour la fabrication.

8. Dispositif de pulvérisation selon une quelconque des revendications précédentes, les trous (13) ayant été formés par perçage au laser des segments à paroi mince (18).

9. Méthode de réalisation d'un dispositif de pulvérisation pour la génération d'un aérosol à déplacement lent, l'aérosol étant généré à partir d'au moins deux jets incidents, et les jets étant formés en forçant un liquide à travers un ensemble d'embout unique en plastique moulé, la méthode étant **caractérisée par** les étapes suivantes :
réalisation d'une pièce unique en plastique moulé comprenant deux ou plusieurs segments à paroi mince (18), chacun étant à peu près carré de façon à présenter une surface projetée substantiellement carrée, chacun des segments à paroi mince (18) étant supporté sur tous les côtés par des segments à paroi épaisse (17, 19), un rapport de forme, défini par le rapport de la longueur d'un côté d'un segment à paroi mince (18) correspondant sur l'épaisseur de paroi du segment à paroi mince (18), étant compris entre 1 et 10 ; et
le perçage au laser d'au moins deux trous opposés (13), chacun des trous (13) s'étendant à travers un segment correspondant des segments à paroi mince (18), chacun des trous (13) mesurant de 5µm à 100µm de diamètre hydraulique (généralement 30µm) et son axe se trouvant à un angle compris entre 55 et 125 degrés (de préférence 90 degrés) par rapport à une surface externe du segment à paroi mince (18) correspondant, les segments à paroi mince (18) étant inclinés de sorte que les surfaces projetées des trous (13) se croisent au moins partiellement du côté de la sortie de l'ensemble d'embout.
